# EUROPEAN PATENT SPECIFICATION

(11) **EP 2 590 614 B1**
(45) Date of publication and mention of the grant of the patent: **01.11.2017**
(21) Application number: 11728891.0
(22) Date of filing: 05.07.2011
(51) Int. Cl.: A61J 3/06, A61M 5/00, F04B 43/12, A61K 9/19, A61K 9/20

(54) **METHOD FOR DOSING A FLUID FORMULATION CONTAINING A MEDICINAL SUBSTANCE**
VERFAHREN ZUR DOSIERUNG EINER FLÜSSIGFORMULIERUNG MIT EINEM MEDIZINISCHEN INHALTSSTOFF
PROCÉDÉ DE DOSAGE D'UNE FORMULATION DE FLUIDE CONTENANT UNE SUBSTANCE MÉDICINALE

(30) Priority: 09.07.2010 US 362860 P; 06.07.2010 EP 10168627
(43) Date of publication of application: 15.05.2013
(73) Proprietor: Intervet International B.V., 5831 AN Boxmeer (NL); Merck Sharp & Dohme B.V., 2031 BN Haarlem (NL)
(72) Inventor: MIDDELBEEK, Hans Almer, NL-5345 PD Oss (NL); JANSSEN, Mari, NL-5340 BH Oss (NL)
(74) Representative: Intervet International B.V.
(86) International application number: PCT/EP2011/061274
(87) International publication number: WO 2012/004239

(56) References cited:
- WO-A1-93/12770
- US-A- 5 382 437
- US-A1- 2003 086 973

## Description

The present invention pertains to a method for dosing a fluid formulation containing a medicinal substance into a mould to form a pharmaceutical unit with a volume of 1000µl or less. In the pharmaceutical industry (both human and veterinary), tablets may be formed by dosing a liquid formulation containing a medicinal substance into a mould, whereafter the liquid formulation is transformed into a solid formulation, e.g. by freezing the liquid, evaporating the solvent, gelling the liquid etc. For forming tablets, a volume of less than 1000 µl is commonly used. Typical amounts are between 100 and 500 µl. This way tablets can be formed for example for direct oral administration or for mixing with a liquid for oral, rectal or parenteral administration. Using a volume above 1000µl leads to very large tablets, not suitable for oral administration and inconvenient for manual handling. Apart from dosing a fluid formulation in order to ultimately form a tablet, the fluid formulation can also be left as a fluid, for example in a vial, syringe or other container for administration or mixing in the form of a fluid. The pharma industry typically uses plunger pumps for secure dosing of specified volumes around 1000µl or less. As is commonly known, plunger pumps are very accurate, leading to typical deviations of the tablet volume below 5% (relative standard deviation), or even below 2%.

A very important disadvantage of plunger pumps however is that they need to be cleaned very regularly because of the high risk of fluid remaining in so-called dead space of the pumping cylinder after dosing. Such remains of the fluid need to be removed regularly to prevent the risk of contamination. For adequate cleaning the plunger pump needs to be disassembled, which takes a significant amount of time.

As is commonly known, peristaltic pumps do not have this problem. A peristaltic pump, (also called "roller pump"), is a type of positive displacement pump used for pumping a variety of fluids. The fluid is contained within a flexible tube typically fitted inside a circular pump casing (though linear peristaltic pumps have been made). A rotor with a number of "rollers" (which term includes "shoes" or "wipers" and other occluding members) attached to the external circumference compresses the flexible tube. As the rotor turns, the part of tube under compression closes (or "occludes") thus forcing the fluid to be pumped to move through the tube. Additionally, as the tube opens to its natural state after the passing of the cam ("restitution" or "resilience") fluid flow is induced to the pump. This process is called peristalsis. Because the only part of the pump in contact with the fluid being pumped is the interior of the tube, it is easy to sterilize and clean the inside surfaces of the pump by simply replacing the used tube with a new one. Furthermore, since there are no moving parts in contact with the fluid, peristaltic pumps are inexpensive to manufacture. Their lack of valves, seals and glands makes them comparatively inexpensive to maintain, and the use of a hose or tube makes for a relatively low-cost maintenance item compared to other pump types. Peristaltic pumps also minimize shear forces experienced by the fluid, which may help to keep colloids and slurry fluids from separating.
At first glance it would therefore seem obvious to use a peristaltic pump for dosing a fluid formulation containing a medicinal substance. However, peristaltic pumps are designed for continuous dosing, and not for dosing small predetermined volumes of liquid (1000 µl or less). Although in principle a peristaltic pump can be controlled to dose a predetermined amount of liquid, it appears that when the dosing volume gets as low as 1000 µl or less, high deviation of the volume (typically as high as 20% relative standard deviation or more; the relative standard deviation, or RSD, being the absolute value of the coefficient of variation, usually expressed as a percentage) is the inherent result. This is unacceptable for tablets for medicinal use, for either human or veterinary application. This explains why in the pharma industry, peristaltic pumps are not used for dosing a fluid formulation into a mould to form a unit with a volume of 1000µl or less.

Surprisingly however, it was found that a peristaltic pump can adequately be used to dose a fluid formulation into a mould (the term "mould" encompasses any element which has a cavity into which the fluid formulation can be dosed; as such, the term "mould" also encompasses a vial, syringe, blister, or any other container) to form a unit with a volume of 1000µl or less, even with a relative standard deviation of 5% or less, when several improvements are implemented comprising placing a spout of the tube of the peristaltic pump adjacent the mould, displacing the roller over a predetermined length of the tube to dose the fluid formulation in the mould, positioning the spout such that when the dosing is finished, at least the open end of the spout is present in the dosed unit, and after dosing removing the spout out of the dosed unit. It is not completely understood why these steps lead to an adequately small relative standard deviation of the dosed volume, but it may depend largely of the end of the spout (for example the end of a needle or any other non-flexible spout) being present in the dosed unit at the end of the dosing stage. Usually it is not recommended to keep the end of a dosing spout in a dosed volume of liquid, but it may be that this is essential, in combination with the other steps, to provide an adequate solution for dosing a predetermined amount of liquid with a volume of 1000µl or less.

The present invention is not restricted to special types of peristaltic pumps. A typical example of a commonly used type of peristaltic pump is a so-called "lower pressure" peristaltic pump, which typically has dry casings and uses rollers as well as non-reinforced, extruded tubing. This class of pump is sometimes called a "tube pump", "tubing pump" or "hose pump". Such a pump typically employs rollers to squeeze the tube. Except for the 360 degree excentric pump design as described below, these pumps typically have a minimum of two rollers 180 degrees apart, and may have as many as 8, or even 12 rollers. Increasing the number of rollers increases the pumping frequency of the fluid at the outlet, and decreases the amplitude of pulsing. The downside to increasing number of rollers it that it proportionately increases number of squeezes, or occlusions, on the tubing for a given cumulative flow through that tube, thereby reducing the tubing life. There are two kinds of roller design in peristaltic pumps. The first is the so-called "fixed occlusion" design, wherein the rollers have a fixed locus as the pump turns, keeping the occlusion constant when the tube is squeezed. This is a simple, yet effective design. The second design is the so-called "spring loaded" design, wherein the rollers are mounted on a spring. This design is a bit more elaborate than the fixed occlusion, but helps overcome variations in the tube wall thickness over a broad range. Irrespective of the variations, the roller imparts the same amount of stress on the tubing that is proportional to the spring constant, making this a constant stress operation. The spring is selected to overcome not only the hoop strength of the tubing, but also the pressure of the pumped fluid.

It is noted that a tablet in the sense of the present invention is a solid dosage form, for example for direct oral, rectal or parenteral administration or for indirect administration for example after mixture with a carrier material, in particular a liquid, for administration in a dissolved or dispersed form. A tablet can be distinguished from powder or fine granules in that a tablet can be individually manually handled. A minimum length size of a tablet is 1 mm, preferably 2 mm, more preferably 4 mm and typically (but not necessarily) between 4 and 20 mm.
A medicinal substance in the sense of the present invention is any substance that can be used to treat a disorder (including diseases), i.e. to aid in preventing, ameliorating or curing the disorder. Such a substance may for example be a chemical or biological compound, such as a natural or synthetic peptide or protein, a (poly-)saccharid or any other organic or inorganic molecule, a dead or alive micro-organism, a dead or alive parasite etc.

In an embodiment the pump has a circular pump casing and the at least one roller is displaced over a predetermined angle. It was found that controlling a predetermined angle is easy to accomplish with commercially available electronic motors, e.g. a stepper type motor.

In another embodiment the tube at the spout has an internal diameter which is smaller than the diameter of a section of the tube being in contact with the roller. Although a spout with a smaller diameter may give rise to pressure build up in the tubing of the pump, and hence dosing variations, it was found that a spout with a diameter which is smaller than a section of the tube being in contact with the roller provides very good results with respect to RSD of the units to be formed. This may be due to the fact that the open end of the spout is present in the unit to be dosed. In a further embodiment the tube itself is segmented, each segment having a different internal diameter. Preferably, the tube comprises a first segment in contact with the roller, a second segment having an internal diameter which is smaller than the internal diameter of the first segment, and a third segment with a spout, the internal diameter of the third segment being smaller than the internal diameter of the second segment. It was surprisingly found that this set-up may give rise to very low RSD levels of the dosed units.

In yet another embodiment the volume of the mould is smaller than the volume of the unit. In prior art methods, typically a mould is chosen that exactly corresponds to the size and shape of the unit to be formed. Surprisingly however, applicant found that a mould may be used that has a volume smaller than that of the unit to be formed. In this embodiment the dosed unit projects from the surface of the element in which the open mould is formed. This set up is possible when the fluid formulation is rapidly solidified when dosed into the mould. If for example the fluid formulation is based on water as a carrier, keeping the mould at a temperature below 0°C may lead to adequate fast solidification. If a gelling agent is present in the fluid formulation, cooling may even be dispensed with. An advantage of this particular embodiment is that the dosed unit, after it has been solidified, can be removed relatively easy from the mould since the contact surface between the unit and mould is small when compared to a unit that is completely enclosed by or sunk in the mould. Another advantage of this embodiment is that there may be provided a discontinuity in the physical appearance of the solidified unit corresponding to the transition site between the mould and the open space above the mould. Since the solid unit to be formed sticks out of the mould, there may be provided a discontinuity in the shape of the unit at the mould entry. Such a discontinuity may be used to distinguish the resulting solid unit from units of a different make (thus being for example an alternative for a company logo, icon or colour), or may be used to provide advantageous mechanical properties. In a preferred embodiment, the volume of the mould is smaller than 50% of the volume of the unit. In this embodiment, more than half of the unit sticks out of the mould. This makes removal of the solidified unit very easy.

In an embodiment the mould has a temperature below a solidification temperature of the formulation, in order to solidify the formulation while present in the mould to form a solid pellet. In this embodiment firstly the mould is filled with the fluid formulation (which wording also covers filling the mould with two or more separate sub-formulations which together form the fluid formulation containing the medicinal substance) and then solidifying the fluid formulation in that mould to form a solid pellet by simply leaving the fluid formulation in the pre-cooled mould, not applying any active shaping tools such that one may end up with an uncompressed frozen pellet having a shape (at the open end of the mould) that is formed merely by gravitational forces and surface tension (a meniscus). It was found that it is particularly advantageous to have the mould at a temperature below the solidification (or freezing) temperature of the formulation upon actual filling the cavity. At first glance this seems a disadvantage: The fluid formulation namely will start to solidify immediately upon contact with the mould, theoretically leading to a solid pellet of a size and shape that does not correspond to the size and shape of the mould, thus leading to an uncontrolled solidification process and thus an unpredictable pellet form. However, applicant found that for a temperature below the solidification temperature of the fluid formulation a filling speed can always be found that is fast enough to counterbalance the immediate solidification of the fluid, simply because the amount of heat present in the flow of fluid formulation may simply counterbalance the extraction of heat by the cold mould, or at least an adequate part of the heat extraction. Overall, the new process is easier to control: the temperature of the mould can be kept at the same level: no cooling-heating cycle has to take place. Moreover, the process is fast. Heat is already being extracted upon filling the mould. Also, there is hardly any risk of loosing fluid from the mould, since the fluid will cool down very fast after entry of the mould and thus will immediately show an increased viscosity.

In a further embodiment the solid pellet is taken out of the mould and dried in a vacuum to obtain a solid tablet. It was found that a frozen pellet may have sufficient mechanical strength to be taken out of the cavity for further processing such as lyophilising. Lyophilising a frozen pellet may lead to a so-called fast disintegrating tablet. To "disintegrate" is to "lose unity and be reduced to fragments". The term "disintegrate" covers dissolution (having fragments at molecular level). Fast-disintegration means disintegration which starts upon contact with a liquid, in particular water at 37°C, and is completed within 60 seconds, preferably within 30 seconds, more preferably within 10 seconds. Fast disintegrating tablets or often also referred to as "orally disintegrating tablets". An overview of such tablets and technologies is provided by Deepak Kaushik, Harish Dureja and T.R. Saini, Maharishi Dayanand University and Shri G.S. Institute of Technology and Science, in "Tablets and Capsules", 30 July 2004). In particular technologies such as Zydis (Cardinal Health, Somerset, NJ, USA) and Lyoc (Laboratoires Farmalyoc, Maisons-Alfort, France) use this known process to from fast-disintegrating tablets.

The invention will now be further explained using the following figures and example.
Figure 1 schematically shows a peristaltic pump and corresponding reservoir and mould.
Figure 2 schematically shows the open end of the pump tubing adjacent the mould.
Figure 3 schematically shows a segmented tubing for the pump.
Figure 4 shows a lyophiliser.
Figure 5 graphically shows the effect of the type of tubing on RSD of the resulting dosed units.

Example 1 describes a method for dosing a fluid formulation according to the present invention.

### Figure 1

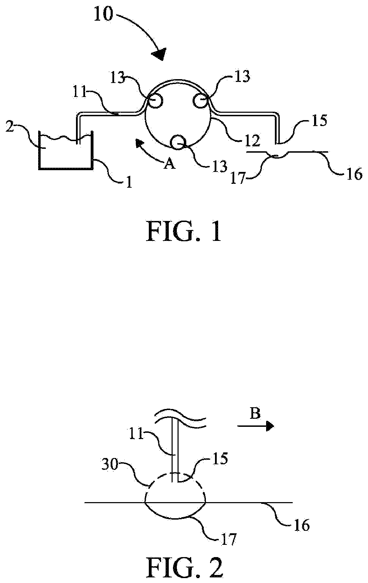

Figure 1 schematically shows a peristaltic pump 10 and corresponding reservoir 1 and mould 17. Pump 10 in this embodiment is of the lower pressure type. This pump exists of circular casing 12 and has three rollers 13 that act to squeeze tube 11 when the rollers rotate in direction A as indicated. By squeezing the tube 11, fluid formulation 2 present in reservoir 2 is transported through the tube in the direction of solid element 16. In this element a mould (cavity) 17 is formed. The open end of the tube (spout) 15 is placed adjacent mould 17 such that a predetermined amount of fluid formulation 2 can be dosed into the mould. In this case the spout has the same internal diameter as the tube 11. By rotating the rollers in direction A over a predetermined angle α, a predetermined volume of the formulation can be transported through the tube 11 into mould 17.

### Figure 2

Figure 2 schematically shows the open end 15 of the pump tubing adjacent the mould 17. In this figure, the dosed unit to be formed is indicated with reference number 30. The end of tube 11, spout 15 is initially placed adjacent the mould such that the fluid formulation will be pumped into mould 17. The spout is placed such that when the unit is completely dosed, the spout is present within the fluid unit as indicated. Surface tension of the fluid formulation will assure that the unit remains intact in the open mould. When the dosing is completed, the tube 11 is removed by displacing tube 11 in direction B.

### Figure 3

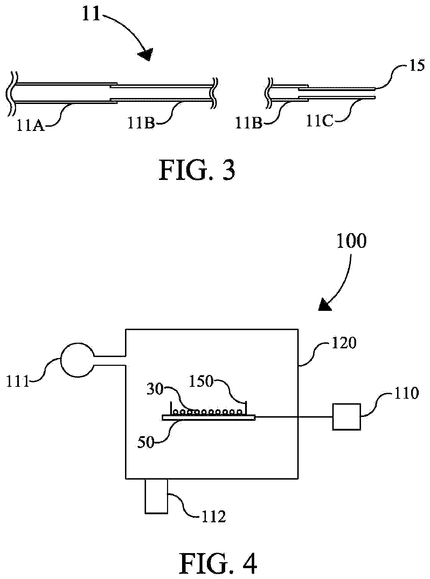

Figure 3 schematically shows a segmented tubing 11 for the pump. In this embodiment the tubing has a first section 11A ("pump section"), which section is adjacent the pump casing and can be squeezed by rollers 13. The tubing has a second section 11B ("transport section") which has a smaller internal diameter. This section extends from the pump and may be as long as 1 to 2 meters typically in order to reach the mould. Just prior to the mould, the tubing has a third section 11C ("spout section"), which on its turn has a smaller internal diameter than section 11B. This last section may be a flexible tubing, but may also be a non-flexible syringe needle. The lasts section comprises the open end 15 of the tubing.

### Figure 4

In figure 4 a lyophiliser (freeze-dry apparatus) is schematically depicted. Such a lyophiliser could for example be the Christ Epsilon 2-12D as available from Salm en Kipp, Breukelen, The Netherlands. The lyophiliser 100 as depicted comprises a housing 120 and one shelve 50. The Epsilon 2-12D itself comprises 4 + 1 shelves, for matters of convenience only one shelves is shown in figure 4. As is commonly known, such a shelves is provided with a heating element (not shown) for even heating of the shelve 50. The heating is controlled by making use of processing unit 110. The housing is connected to a pump unit 111 for providing adequate low pressure within the housing 120. The interior of the housing can be cooled to a temperature as low as -60°C by using cooling unit 112, in particular containing a condenser (in fact, it is the condenser that is kept at about -60°C, which acts as a driving force for condensation of sublimated ice). Placed on the shelve is a container 150. This container is made of a heat conducting material, in this case carbon black filled polyethyleneterephtalate. The container is in a heat conducting contact with the shelve 50. In the shown arrangement, the container is filled with a monolayer of frozen pellets 30. By heating the shelve, the particles receive heat via the heated bottom and side walls of the container, such that the frozen liquid can sublimate and the pellet can be dried to from a (fast-disintegrating) tablet.

### Example 1

With a pumping set-up corresponding to the schematic set-up as depicted in Figure 1 (a rotating lower pressure hose pump), a water based fluid formulation was dosed into an open mould (of a type as shown in figure 2). At the end of dosing a predetermined volume, the spout 15 was in each case present in the fluid unit. Right after the dosing of a unit was completed, the spout was removed from the unit by horizontal displacement. Dosing volumes of 50µl, 100µl, 250µl, 500µl, 700µl and 1000µl were applied, where appropriate with different tubing. For each dosing volume and chosen tubing, at least 20 pellets were made. Of these pellets the relative standard deviation (RSD in %) of the volume was determined.

The following tubings were used:
- 2.38 mm internal diameter (non-segmented)
- 1.52 mm internal diameter (non-segmented)
- 1.29 mm internal diameter (non-segmented)
- 1.02 mm internal diameter (non-segmented)
- 0.63 mm internal diameter (non-segmented)
- 2.54/1.5/0.9 mm (pump section/transport section/spout section)
- 2.54/1.5/1.2 mm (pump section/transport section/spout section)

Beforehand, one would expect that a larger dosing volume leads to a lower RSD. Also, one would expect that a smaller tubing diameter would lead to a lower RSD, although for each set-up this has to be confirmed experimentally.

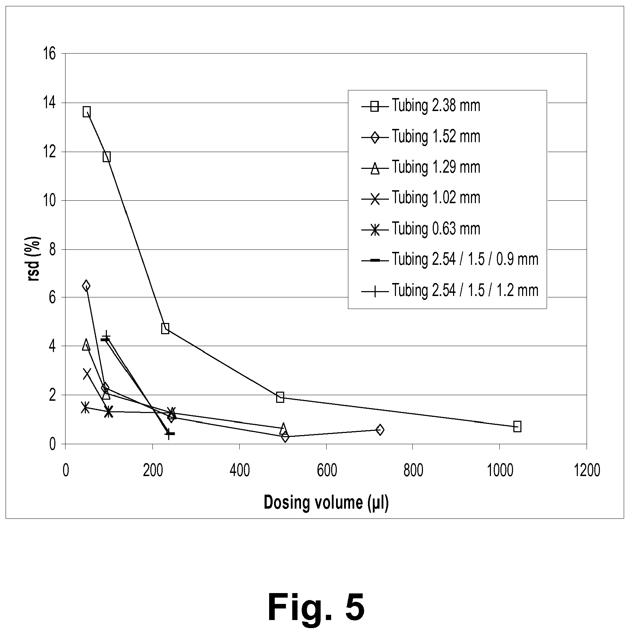

The results are given in Figure 5. From this figure it indeed appears that a larger dosing volume corresponds to a lower RSD. What is surprising however is that with a simple peristaltic pump, one can reach typical values of the RSD as low as 5 or even 2%, even for the dosed volumes below 1000µl. Indeed, when the dosing volume is below 100µl, in this set-up one needs to turn to tubing with an internal diameter around 1 mm or smaller. From a practical point of view however, this is no disadvantage at all.

What came as a second surprise is that when using a segmented tube, a pump tube can be used that still has a relatively large diameter (2.54 mm!), and still RSD values below 5% can be reached, even for a dosing volume as small as 100µl. This is highly surprising since one would expect that the dosing accuracy depends largely on the actual squeezing contact between the rollers and tube at the pump. It was however found that a far higher accuracy than expected given the diameter of the tube at the pump section can be provided when using down-stream tube sections with a smaller diameter.

## Claims

1. Method for dosing a fluid formulation containing a medicinal substance into a mould to form a pharmaceutical unit with a volume of 1000µl or less, using a peristaltic pump comprising a flexible tube for guiding the fluid formulation to the mould and at least one roller to compress the tube to induce a flow of the fluid formulation through the tube, the method comprising:
- placing a spout of the tube adjacent the mould,
- displacing the roller over a predetermined length of the tube to dose the fluid formulation in the mould,
- positioning the spout such that when the dosing is finished, at least the open end of the spout is present in the dosed unit, and after dosing
- removing the spout out of the dosed unit.

2. Method according to claim 1, **characterised in that** the pump has a circular pump casing and the at least one roller is displaced over a predetermined angle.

3. Method according to any of the preceding claims, **characterised in that** the tube at the spout has an internal diameter which is smaller than the diameter of a section of the tube being in contact with the roller.

4. Method according to claim 3, **characterised in that** the tube is segmented, each segment having a different internal diameter.

5. Method according to claim 4, **characterised in that** the tube comprises a first segment in contact with the roller, a second segment having an internal diameter which is smaller than the internal diameter of the first segment, and a third segment with a spout, the internal diameter of the third segment being smaller than the internal diameter of the second segment.

6. Method according to any of the preceding claims, **characterised in that** the volume of the mould is smaller than the volume of the unit.

7. A method according to any of the preceding claims, **characterised in that** the mould has a temperature below a solidification temperature of the formulation, in order to solidify the formulation while present in the mould to form a solid pellet.

8. A method according to claim 7, **characterised in that** the solid pellet is taken out of the mould and dried in a vacuum to obtain a solid tablet.

## Patentansprüche

1. Verfahren zur Dosierung einer Flüssigformulierung, die eine medizinische Substanz enthält, in eine Form zur Bildung einer pharmazeutischen Einheit mit einem Volumen von 1000 µl oder weniger unter Verwendung einer Peristaltikpumpe, die einen flexiblen Schlauch zum Leiten der Flüssigformulierung zur Form und mindestens eine Walze zum Zusammendrücken des Schlauchs, um eine Strömung der Flüssigformulierung durch den Schlauch auszulösen, umfasst, wobei das Verfahren Folgendes umfasst:
- Platzieren einer Auslauftülle des Schlauchs neben der Form,
- Verschieben der Walze über eine vorbestimmte Länge des Schlauchs zur Dosierung der Flüssigformulierung in der Form,
- Positionieren der Auslauftülle, so dass am Ende der Dosierung mindestens das offene Ende der Auslauftülle in der dosierten Einheit vorhanden ist, und nach der Dosierung
- Entfernen der Auslauftülle aus der dosierten Einheit.

2. Verfahren nach Anspruch 1, **dadurch gekennzeichnet, dass** die Pumpe ein kreisförmiges Pumpengehäuse aufweist und die mindestens eine Walze über einen vorbestimmten Winkel verschoben wird.

3. Verfahren nach einem der vorhergehenden Ansprüche, **dadurch gekennzeichnet, dass** der Schlauch an der Auslauftülle einen Innendurchmesser aufweist, der kleiner als der Durchmesser eines Abschnitts des Schlauchs in Kontakt mit der Walze ist.

4. Verfahren nach Anspruch 3, **dadurch gekennzeichnet, dass** der Schlauch segmentiert ist, wobei jedes Segment einen unterschiedlichen Innendurchmesser aufweist.

5. Verfahren nach Anspruch 4, **dadurch gekennzeichnet, dass** der Schlauch ein erstes Segment in Kontakt mit der Walze, ein zweites Segment mit einem Innendurchmesser, der kleiner als der Innendurchmesser des ersten Segments ist, und ein drittes Segment mit einer Auslauftülle umfasst, wobei der Innendurchmesser des dritten Segments kleiner als der Innendurchmesser des zweiten Segments ist.

6. Verfahren nach einem der vorhergehenden Ansprüche, **dadurch gekennzeichnet, dass** das Volumen der Form kleiner als das Volumen der Einheit ist.

7. Verfahren nach einem der vorhergehenden Ansprüche, **dadurch gekennzeichnet, dass** die Form eine Temperatur unter der Erstarrungstemperatur der Formulierung aufweist, damit die Formulierung zur Bildung eines festen Pellets erstarrt, während sie sich in der Form befindet.

8. Verfahren nach Anspruch 7, **dadurch gekennzeichnet, dass** das feste Pellet aus der Form genommen und in einem Vakuum getrocknet wird, um eine feste Tablette zu erhalten.

## Revendications

1. Procédé d'introduction dosée d'une formulation de fluide contenant une substance médicinale dans un moule pour former une unité pharmaceutique ayant un volume de 1 000 µl ou moins, à l'aide d'une pompe péristaltique comprenant un tube souple pour le guidage de la formation de fluide dans le moule et au moins un rouleau pour la compression du tube en vue d'induire un écoulement de la formulation de fluide à travers le tube, le procédé comprenant :
- le placement d'un bec du tube adjacent au moule,
- le déplacement du rouleau sur une longueur prédéterminée du tube pour l'introduction dosée de la formulation de fluide dans la moule,
- le positionnement du bec de telle sorte que, lorsque l'introduction dosée est terminée, au moins l'extrémité ouverte du bec est présente dans l'unité dosée, et après l'introduction dosée,
- le retrait du bec de l'unité dosée.

2. Procédé selon la revendication 1, **caractérisé en ce que** la pompe comporte un corps de pompe circulaire et ledit au moins un rouleau est déplacé sur un angle prédéterminé.

3. Procédé selon l'une quelconque des revendications précédentes, **caractérisé en ce que** le tube, au niveau du bec, a un diamètre interne qui est inférieur au diamètre d'une section du tube en contact avec le rouleau.

4. Procédé selon la revendication 3, **caractérisé en ce que** le tube est segmenté, chaque segment ayant un différent diamètre interne.

5. Procédé selon la revendication 4, **caractérisé en ce que** le tube comprend un premier segment en contact avec le rouleau, un deuxième segment ayant un diamètre interne qui est inférieur au diamètre interne du premier segment, et un troisième segment pourvu d'un bec, le diamètre interne du troisième segment étant inférieur au diamètre interne du deuxième segment.

6. Procédé selon l'une quelconque des revendications précédentes, **caractérisé en ce que** le volume du moule est inférieur au volume de l'unité.

7. Procédé selon l'une quelconque des revendications précédentes, **caractérisé en ce que** le moule a une température inférieure à une température de solidification de la formulation, afin de solidifier la formulation tandis qu'elle se trouve dans le moule pour former un granulé solide.

8. Procédé selon la revendication 7, **caractérisé en ce que** le granulé solide est retiré du moule et séché sous vide pour produire un comprimé solide.
